(19) Europäisches Patentamt
European Patent Office
Office européen des brevets

(11) Veröffentlichungsnummer : **0 379 098 B1**

(12) **EUROPÄISCHE PATENTSCHRIFT**

(45) Veröffentlichungstag der Patentschrift : **22.07.92 Patentblatt 92/30**

(51) Int. Cl.$^5$ : **C07C 317/46,** C07C 323/56, A01N 37/10

(21) Anmeldenummer : **90100658.5**

(22) Anmeldetag : **13.01.90**

# (54) Schwefelhaltige Acrylsäureester und diese enthaltende Fungizide.

Die Akte enthält technische Angaben, die nach dem Eingang der Anmeldung eingereicht wurden und die nicht in dieserPatentschrift enthalten sind.

(30) Priorität : **20.01.89 DE 3901607**

(43) Veröffentlichungstag der Anmeldung : **25.07.90 Patentblatt 90/30**

(45) Bekanntmachung des Hinweises auf die Patenterteilung : **22.07.92 Patentblatt 92/30**

(84) Benannte Vertragsstaaten : **AT BE CH DE DK ES FR GB GR IT LI NL SE**

(56) Entgegenhaltungen :
**EP-A- 0 226 917**
**EP-A- 0 278 595**
**EP-A- 0 299 694**

(73) Patentinhaber : **BASF Aktiengesellschaft**
**Carl-Bosch-Strasse 38**
**W-6700 Ludwigshafen (DE)**

(72) Erfinder : **Wingert, Horst, Dr.**
**D 3,4**
**W-6800 Mannheim 1 (DE)**
Erfinder : **Sauter, Hubert, Dr.**
**Neckarpromenade 20**
**W-6800 Ludwigshafen (DE)**
Erfinder : **Schuetz, Franz, Dr.**
**Budapester Strasse 45**
**W-6700 Ludwigshafen (DE)**
Erfinder : **Wenderoth, Bernd, Dr.**
**Schwalbenstrasse 26**
**W-6840 Lampertheim (DE)**
Erfinder : **Brand, Siegbert, Dr.**
**Hegelstrasse 39**
**W-6940 Weinheim (DE)**
Erfinder : **Mueller, Bernd, Dr.**
**Jean-Ganss-Strasse 21**
**W-6710 Frankenthal (DE)**
Erfinder : **Roehl, Franz, Dr.**
**Karl-Otto-Braun-Strasse 8**
**W-6700 Ludwigshafen (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

EP 0 379 098 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Acrylester-Derivate, ihre Herstellung, diese enthaltende Fungizide und ihre Verwendung als Fungizide.

Es ist bekannt, Acrylsäureester wie zum Beispiel den α-(2-[4-Chlorphenylthiomethyl]phenyl)-β-methoxyacrylsäure-methylester (EP 226 917) oder den α-(2-[3-Chlorphenylthiomethyl]phenyl)-β-methoxyacrylsäure-methylester (EP 278 595) als Fungizide zu benutzen.

Es wurde nun gefunden, daß substituierte Acrylester-Derivate der allgemeinen Formel

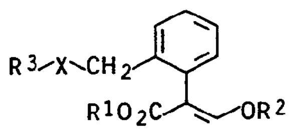

I

in der

$R^1$, $R^2$

Wasserstoff oder $C_1$-$C_5$-Alkyl bedeutet,

X

S bedeutet,

$R^3$

unsubstituiertes Naphthyl oder phenanthrenyl oder substituiertes Phenyl, Naphthyl oder phenanthrenyl bedeutet, und diese Reste substituiert sind durch Fluor, Brom, Cyan, Nitro, Formyl, $C_1$-$C_4$-Alkoxy, $C_1$-$C_2$-Halogenalkyl, Aryl, $C_1$-$C_4$-Alkoxycarbonyl

und die Verbindungen der Formel I, in der X = S und $R^3$ = ein Phenylrest ist, der durch 2-Chlor, 2,5-Dichlor, 2,4-Dimethyl substituiert ist in ihrer fungiziden Wirkung den oben erwähnten bekannten Verbindungen überlegen sind.

Die in der allgemeinen Formel I aufgeführten Reste können beispielsweise folgende Bedeutung haben: $R^1$ und $R^2$ sind gleich oder verschieden und stehen z. B. für Wasserstoff, $C_1$-$C_5$-Alkyl, Methyl, Ethyl, Propyl, i-propyl, Butyl, Pentyl; Methyl wird bevorzugt.

Die Reste $R^3$ können z.B. folgende Substituenten tragen:

$C_1$-$C_2$-Halogenalkyl (z. B. Difluormethyl, Trifluormethyl, Chlormethyl, Dichlormethyl, Trichlormethyl, Pentafluorethyl), $C_1$-$C_4$-Alkoxy (z. B. Methoxy, Ethoxy, n- oder iso-Propoxy; n-, iso-, sec.- oder tert.-Butoxy), Aryl (z. B. Phenyl, Naphthyl), $C_1$-$C_4$-Alkoxycarbonyl (z. B. Methoxycarbonyl, Ethoxycarbonyl, Propoxycarbonyl, Butoxycarbonyl).

Die neuen Verbindungen der Formel I können aufgrund der C=C-Doppelbindung sowohl als E- als auch als Z-Isomere vorliegen. Sowohl die einzelnen isomeren Verbindungen als auch ihre Gemische werden von der Erfindung umfaßt und sind als Fungizide brauchbar.

Die neuen Verbindungen der allgemeinen Formel I mit X = S lassen sich beispielsweise herstellen, indem man Thiole der Formel (II) mit einem Benzylbromid der allgemeinen Formel III umsetzt.

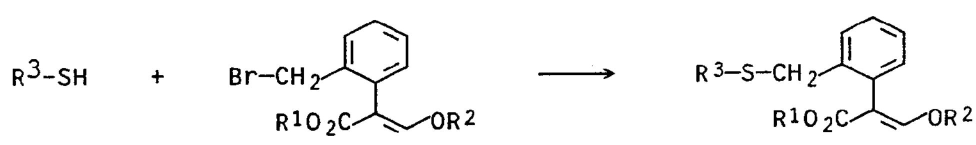

II III I

$R^1$, $R^2$, $R^3$ haben die Bedeutungen wie im Anspruch 1.

Die Umsetzungen zu den Verbindungen der Formel I können z. B. in einem inerten Lösungs- oder Verdünnungsmittel (z. B. Aceton, Acetonitril, Dimethylsulfoxid, Dioxan, Dimethylformamid, N-Methylpyrrolidon, N,N′-Dimethylpropylenharnstoff oder Pyridin) unter Verwendung einer Base (z. B. Natriumcarbonat, Kaliumcarbonat) durchgeführt werden. Außerdem kann es von Vorteil sein, der Reaktionsmischung einen Katalysator wie z. B. Tris-(3,6-dioxoheptyl)-amin zuzusetzen (J. Org. Chem. 50 (1985) 3717).

Alternativ kann auch.so vorgegangen werden, daß man die Verbindungen der allgemeinen Formel II zunächst mit einer Base (z. B. Natriumhydroxid oder Kaliumhydroxid) in die entsprechenden Natrium-oder Kaliumsalze überführt und diese dann in einem inerten Lösungs-oder Verdünnungsmittel (z. B. Dimethylformamid) mit dem Benzylbromid der Formel III zu den entsprechenden Verbindungen der allgemeinen Formel I umsetzt.

Die Thiole der allgemeinen Formel $R^3$-SH ($R^3$ hat die oben angegebene Bedeutung) sind entweder bekannt oder können durch Verfahren analog zu bekannten Verfahren hergestellt werden. Entsprechende Herstellverfahren sind z. B. beschrieben in Houben-Weyl, Methoden der organischen Chemie VI/3, 54 ff (1965).

α-Brommethylphenyl-acrylester der allgemeinen Formel III sind bekannt aus DE-35 19 280, DE-35 45 318 und DE-35 45 319.

Beispiele

Die nachstehenden Vorschriften erläutern die Herstellung der neuen Wirkstoffe.

Beispiel 1

α-(2-Naphthylthiomethyl-phenyl)-β-methoxyacrylsäure-methylester (Verbindung 2, Tabelle 1)

8 g α-(2-Brommethyl-phenyl)-β-methoxyacrylsäure-methylester, 4,5 g 2-Thionaphthol, 4,4 g Kaliumcarbonat und 100 mg Kaliumiodid werden in 200 ml Aceton 20 Stunden unter Rückfluß erhitzt. Man gibt 200 ml Wasser zu und extrahiert 3x mit Methylenchlorid. Die organischen Phasen werden über $Na_2SO_4$ getrocknet und eingeengt. Das verbleibende Öl wird an Kieselgel mit n-Hexan/Essigester-Gemisch chromatographiert. man erhält 8,19 g schwach gelbe Kristalle vom Fp. 52-58 °C.

In entsprechender Weise lassen sich die folgenden Verbindungen herstellen:

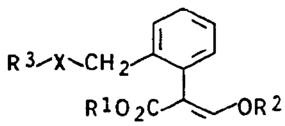

I

Tabelle 1: X = S

| Nr. | $R^3$ | $R^1$ | $R^2$ | IR ($cm^{-1}$) (Fp.) |
|---|---|---|---|---|
| 1 | 1-Naphthyl | $CH_3$ | $CH_3$ | |
| 2 | 2-Naphthyl | $CH_3$ | $CH_3$ | 52- 58 °C |
| 3 | 3-Phenanthrenyl | $CH_3$ | $CH_3$ | |
| 4 | 2-Chlor-phenyl | $CH_3$ | $CH_3$ | 97- 99 °C |
| 5 | 2-Brom-phenyl | $CH_3$ | $CH_3$ | 121-127 °C |
| 6 | 3-Brom-phenyl | $CH_3$ | $CH_3$ | 84- 86 °C |
| 7 | 4-Brom-phenyl | $CH_3$ | $CH_3$ | 84- 91 °C |
| 8 | 2-Fluor-phenyl | $CH_3$ | $CH_3$ | 84- 88 °C |
| 9 | 3-Fluor-phenyl | $CH_3$ | $CH_3$ | 77- 82 °C |
| 10 | 4-Fluor-phenyl | $CH_3$ | $CH_3$ | 50- 54 °C |
| 24 | 2-Methoxy-phenyl | $CH_3$ | $CH_3$ | 77- 85 °C |
| 25 | 3-Methoxy-phenyl | $CH_3$ | $CH_3$ | 52- 58 °C |
| 26 | 4-Methoxy-phenyl | $CH_3$ | $CH_3$ | 70- 72 °C |
| 27 | 2-Trifluormethyl-phenyl | $CH_3$ | $CH_3$ | |
| 28 | 3-Trifluormethyl-phenyl | $CH_3$ | $CH_3$ | |
| 29 | 4-Trifluormethyl-phenyl | $CH_3$ | $CH_3$ | |
| 30 | 4-Formyl-phenyl | $CH_3$ | $CH_3$ | |
| 31 | 2-Nitro-phenyl | $CH_3$ | $CH_3$ | |
| 32 | 3-Nitro-phenyl | $CH_3$ | $CH_3$ | |
| 33 | 4-Nitro-phenyl | $CH_3$ | $CH_3$ | |
| 34 | 2,5-Dichlor-phenyl | $CH_3$ | $CH_3$ | 69- 73 °C |

Tabelle 1: X = S (Fortsetzung)

| Nr. | $R^3$ | $R^1$ | $R^2$ | IR ($cm^{-1}$) (Fp.) |
|---|---|---|---|---|
| 54 | 3,5-Bistrifluormethyl-phenyl | $CH_3$ | $CH_3$ | |
| 57 | 2,4-Dimethyl-phenyl | $CH_3$ | $CH_3$ | 57- 62 °C |
| 65 | 2,5-Dimethoxy-phenyl | $CH_3$ | $CH_3$ | |
| 66 | 3,4-Dimethoxy-phenyl | $CH_3$ | $CH_3$ | |
| 68 | 2-Methoxycarbonyl-phenyl | $CH_3$ | $CH_3$ | |
| 69 | 2-Ethoxycarbonyl-phenyl | $CH_3$ | $CH_3$ | |
| 70 | 2-Propoxycarbonyl-phenyl | $CH_3$ | $CH_3$ | |
| 71 | 2-Butoxycarbonyl-phenyl | $CH_3$ | $CH_3$ | |
| 72 | 2-Cyano-phenyl | $CH_3$ | $CH_3$ | |
| 73 | 3-Cyano-phenyl | $CH_3$ | $CH_3$ | |
| 74 | 4-Cyano-phenyl | $CH_3$ | $CH_3$ | |

Die neuen Verbindungen zeichnen sich, allgemein ausgedrückt, durch eine hervorragende Wirksamkeit

gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zübereitungen sind:

I. Man vermischt 90 Gew.-Teile der Verbindung Nr. 2 (Tab. 1) mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 4 (Tab. 1) werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 25 (Tab. 1) werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 34 (Tab. 1) werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 (Tab. 1) werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 4 (Tab. 1) werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 25 (Tab. 1) werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 34 (Tab. 1) werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 (Tab. 1) werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden α-(2-[3-Chlorphenylthiomethyl)-phenyl)-β-methoxyacrylsäuremethylester (A) - bekannt aus EP 278 595 - und α-(2-[4-Chlorphenylthiomethyl)-phenyl)-β-methoxyacrylsäuremethylester (B) - bekannt aus Ep 226 917 - verwendet.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbeleges wurden die versuchspflanzen im Gewächshaus bei Temperaturen zwischen 20 und 22°C und 65 und 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 4 und 34 (Tab. 1) bei der Anwendung als 0,025 %ige (Gew.%) Spritzbrühe eine bessere fungizide Wirkung zeigen (94 %) als die bekannten Vergleichswirkstoffe A (65 %) und B (80 %).

Anwendungsbeispiel 2

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inoculiert. Anschließend wurden die Versuchspflanzen iin Klimakammern bei 22 - 24°C und 95 - 99 % relativer Luftfeuchtigkeit aufgestellt. Nach 6 Tagen wurde das Ausmaß des Krankheitsbefalls ermittelt.

Das Ergebnis zeigt, daß die Wirkstoffe 2, 4, 25 und 34 (Tab. 1) bei der Anwendung als 0,05 %ige Spritzbrühe eine bessere fungizide Wirkung zeigen (94 %) als die bekannten Vergleichswirkstoffe A (65 %) und B (55 %).

## Patentansprüche

1. Acrylsäureester der Formel

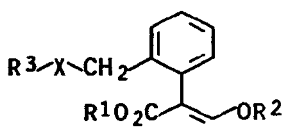

I

in der
$R^1$, $R^2$
Wasserstoff oder $C_1$-$C_5$-Alkyl bedeutet,
X
S bedeutet,
$R^3$
unsubstituiertes Naphthyl oder Phenanthrenyl oder substituiertes Phenyl, Naphthyl oder Phenanthrenyl bedeutet, und diese Reste substituiert sind durch Fluor, Brom, Cyan, Nitro, Formyl, $C_1$-$C_4$-Alkoxy, $C_1$-$C_2$-Halogenalkyl, Aryl, $C_1$-$C_4$-Alkoxycarbonyl
und die Verbindungen der Formel I, in der X = S und $R^3$ = ein Phenylrest ist, der durch 2-Chlor, 2,5-Dichlor, 2,4-Dimethyl substituiert ist.

2. Verfahren zur Herstellung von Acrylsäureestern der Formel I gemäß Anspruch 1, dadurch gekennzeichnet, daß man ein Thiol der allgemeinen Formel

$R^3$-SH (II)

in der $R^3$ die in Anspruch 1 genannte Bedeutung hat, mit einem Benzylhalogenid der allgemeinen Formel III umsetzt,

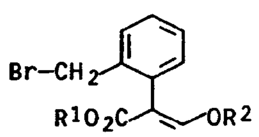

(III)

in der $R^1$ und $R^2$ die in Anspruch 1 genannte Bedeutung hat.

3. Verfahren zur Bekämpfung von Pilzen, dadurch gekennzeichnet, daß man die Pilze oder die vom Pilzbefall bedrohten Materialien, Pflanzen, Saatgüter oder den Erdboden mit einer fungizid wirksamen Menge eines Acrylsäureesters der Formel I behandelt,

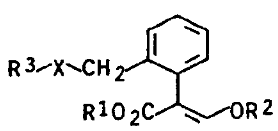

I

in der

$R^1$, $R^2$

Wasserstoff oder $C_1$-$C_5$-Alkyl bedeutet,

X

S bedeutet,

$R^3$

unsubstituiertes Naphthyl oder Phenanthrenyl oder substituiertes Phenyl, Naphthyl oder Phenanthrenyl bedeutet, und diese Reste substituiert sind durch Fluor, Brom, Cyan, Nitro, Formyl, $C_1$-$C_4$-Alkoxy, $C_1$-$C_2$-Halogenalkyl, Aryl, $C_1$-$C_4$-Alkoxycarbonyl

und die Verbindungen der Formel 1, in der X = S und $R^3$ = ein Phenylrest ist, der durch 2-Chlor, 2,5-Dichlor, 2,4-Dimethyl substituiert ist.

4. Fungizid, enthaltend einen inerten Trägerstoff und eine fungizid wirksame Menge eines Acrylsäueresters der Formel I

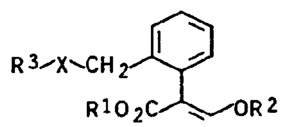

I

in der

$R^1$, $R^2$

Wasserstoff oder $C_1$-$C_5$-Alkyl bedeutet,

X

S bedeutet,

$R^3$

unsubstituiertes Naphthyl oder Phenanthrenyl oder substituiertes Phenyl, Naphthyl oder Phenanthrenyl bedeutet, und diese Reste substituiert sind durch Fluor, Brom, Cyan, Nitro, Formyl, $C_1$-$C_4$-Alkoxy, $C_1$-$C_2$-Halogenalkyl, Aryl, $C_1$-$C_4$-Alkoxycarbonyl

und die Verbindungen der Formel 1, in der X = S und $R^3$ = ein Phenylrest ist, der durch 2-Chlor, 2,5-Dichlor, 2,4-Dimethyl substituiert ist.

5. Verfahren zur Herstellung eines fungiziden Mittels, dadurch gekennzeichnet, daß man eine oder mehrere Verbindungen der Formel I gemäß Anspruch 1 mit einem festen öder flüssigen Trägerstoff sowie gegebenenfalls mit einem oder mehreren oberflächenaktiven Mitteln mischt.

6. Verbindung gemäß Anspruch 1, dadurch gekennzeichnet, daß $R^1$ und $R^2$Methyl, X den Rest S und $R^3$ 2-Naphthyl bedeutet.

7. Verbindung gemäß Anspruch 1, dadurch gekennzeichnet, daß $R^1$ und $R^2$ Methyl, X den Rest S und $R^3$ 2-Chlorphenyl bedeutet.

8. Verbindung gemäß Anspruch 1, dadurch gekennzeichnet, daß $R^1$ und $R^2$ Methyl, X den Rest S und $R^3$ 2,5-Dichlorphenyl bedeutet.

9. Verbindung gemäß Anspruch 1, dadurch gekennzeichnet, daß $R^1$ und $R^2$ Methyl, X den Rest S und $R^3$ 2-Bromphenyl bedeutet.

10. Verbindung gemäß Anspruch 1, dadurch gekennzeichnet, daß $R^1$ und $R^2$ Methyl, X den Rest S und $R^3$ 3-Bromphenyl bedeutet.

## Claims

1. An acrylate of the formula

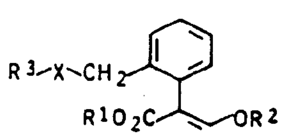

I

where

$R^1$ and $R^2$

are each hydrogen or $C_1$-$C_5$-alkyl,
X
is S and
$R^3$
is unsubstituted naphthyl or phenanthrenyl or substituted phenyl, naphthyl or phenanthrenyl, and these radicals are substituted by fluorine, bromine, cyano, nitro, formyl, $C_1$-$C_4$-alkoxy, $C_1$- or $C_2$-haloalkyl, aryl or $C_1$-$C_4$-alkoxycarbonyl,
and the compounds of the formula I in which X is S and $R^3$ is phenyl which is substituted by 2 -chloro, 2, 5-dichloro or 2, 4 -dimethyl.

2. Process for the preparation of an acrylate of formula I as claimed in claim 1, wherein a thiol of the formula

$R^3$-SH (II)

where $R^3$ has the meanings stated in claim 1, is reacted with a benzyl halide of the formula III

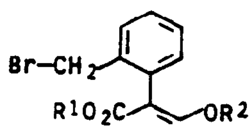

(III)

where $R^1$ and $R^2$ have the meanings stated in claim 1.

3. A method for controlling fungi, wherein the fungi or the materials, plants or seeds threatened by fungal attack or the soil is or are treated with a fungicidal amount or an acrylate of the formula I

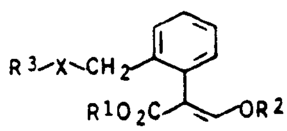

I

where
$R^1$ and $R^2$
are each hydrogen or $C_1$-$C_5$-alkyl,
X
is S and
$R^3$
is unsubstituted naphthyl or phenanthrenyl or substituted phenyl, naphthyl or phenanthrenyl, and these radicals are substituted by fluorine, bromine, cyano, nitro, formyl, $C_1$-$C_4$-alkoxy, $C_1$ or $C_2$-haloalkyl, aryl or $C_1$-$C_4$-alkoxycarbonyl,
and the compounds of the formula I in which X is S and $R^3$ is phenyl which is substituted by 2-chloro, 2, 5-dichloro or 2,4-dimethyl.

4. A fungicide containing an inert carrier and a fungicidal amount of an acrylate of the formula I

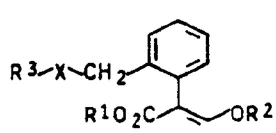

I

where
$R^1$ and $R^2$
are each hydrogen or $C_1$-$C_5$-alkyl,
X
is S and
$R^3$
is unsubstituted naphthyl or phenanthrenyl or substituted phenyl, naphthyl or phenanthrenyl, and these radicals are substituted by fluorine, bromine, cyano, nitro, formyl, $C_1$-$C_4$-alkoxy, $C_1$- or $C_2$-haloalkyl, aryl or $C_1$-$C_4$-alkoxycarbonyl,
and the compounds of the formula I in which X is S and $R^3$ is phenyl which is substituted by 2-chloro, 2,5-dichloro

or 2,4-dimethyl.

5. A process for the preparation of a fungicide, wherein one or more compounds of the formula I as claimed in claim 1 are mixed with a solid or liquid carrier and, if required, with one or more surfactants.

6. A compound as claimed in claim 1, wherein $R^1$ and $R^2$ are each methyl, X is S and $R^3$ is 2-naphthyl.

7. A compound as claimed in claim 1, wherein $R^1$ and $R^2$ are each methyl, X is S and $R^3$ is 2-chlorophenyl.

8. A compound as claimed in claim 1, wherein $R^1$ and $R^2$ are each methyl, X is S and $R^3$ is 2,5-dichlorophenyl.

9. A compound as claimed in claim 1, wherein $R^1$ and $R^2$ are each methyl, X is S and $R^3$ is 2-bromophenyl.

10. A compound as claimed in claim 1, wherein $R^1$ and $R^2$ are each methyl, X is S and $R^3$ is 3-bromophenyl.

## Revendications

1. Esters d'acide acrylique de formule

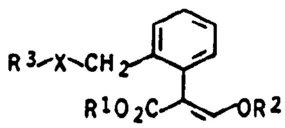

(I)

dans laquelle
$R^1$ et $R^2$
représentent hydrogène ou alkyle en $C_1$-$C_5$,
X
représente S,
$R^3$
représente naphtyle ou phénanthrénylé non substitués ou phényle, naphtyle ou phénanthrénylé substitués, ces restes étant substitués par fluor , brome, cyano, nitro, formyle, alcoxy en $C_1$-$C_4$, halogènalkyle en $C_1$-$C_2$, aryle, alcoxy-$C_1$-$C_4$-carbonyle,
et les composés de formule I dans lesquels X = S et $R^3$ = reste phényle substitué par 2-chlore, 2,5-dichlore, 2,4-diméthyle.

2. Procédé de préparation d'esters d'acide acrylique de formule I, selon la revendication 1, caractérisé par le fait que l'on met à réagir un thiol de formule générale

$$R^3 - SH \qquad (II)$$

dans laquelle $R^3$ a la signification donnée dans la revendication 1, avec un halogénure de benzyle de formule générale III

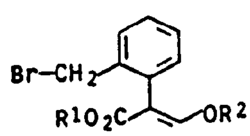

(III)

dans laquelle $R^1$ et $R^2$ ont les significations données dans la revendication 1.

3. Procédé de lutte contre les champignons, caractérisé par le fait que l'on traite les champignons ou le matériaux, plantes, semences ou le sol menacés par l'attaque par les champignons, avec une quantité efficace du point de vue fongicide d'un ester d'acide acrylique de formule I

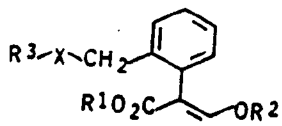

(I)

dans laquelle
$R^1$ et $R^2$
représentent hydrogène ou alkyle en $C_1$-$C_5$,

X
représente S,
$R^3$
représente naphtyle ou phénanthrényle non substitués ou phényle, naphtyle ou phénanthrényle substitués, ces restes étant substitués par fluor , brome, cyano, nitro, formyle, alcoxy en $C_1$-$C_4$, halogènalkyle en $C_1$-$C_2$, aryle, alcoxy-$C_1$-$C_4$-carbonyle,
et les composés de formule I dans lesquels X = S et $R^3$ = reste phényle substitué par 2-chlore, 2,5-dichlore, 2,4-diméthyle.

4. Fongicide contenant un véhicule inerte et une quantité efficace du point de vue fongicide d'un ester d'acide acrylique de formule I

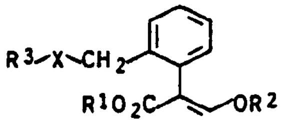

(I)

dans laquelle
$R^1$ et $R^2$
représentent hydrogène ou alkyle en $C_1$-$C_5$,
X
représente S,
$R^3$
représente naphtyle ou phénanthrényle non substitués ou phényle, naphtyle ou phénanthrényle substitués, ces restes étant substitués par fluor , brome, cyano, nitro, formyle, alcoxy en $C_1$-$C_4$, halogènalkyle en $C_1$-$C_2$, aryle, alcoxy-$C_1$-$C_4$-carbonyle,
et les composés de formule I dans lesquels X = S et $R^3$ = reste phényle substitué par 2-chlore, 2,5-dichlore, 2,4-diméthyle.

5. Procédé de préparation d'un agent fongicide, caractérisé par le fait que l'on mélange un ou plusieurs composés de formule I, selon la revendication 1, avec un véhicule solide ou liquide et éventuellement avec un ou plusieurs agents tensio-actifs.

6. Composé selon la revendication 1, caractérisé par le fait que $R^1$ et $R^2$ représentent méthyle, X étant S et $R^3$ 2-naphtyle.

7. Composé selon la revendication 1, caractérisé par le fait que $R^1$ et $R^2$ représentent méthyle, X étant S et $R^3$ représentant 2-chlorophényle.

8. Composé selon la revendication 1, caractérisé par le fait que $R^1$ et $R^2$ représentent méthyle, X étant S et $R^3$ 2,5-dichlorophényle.

9. Composé selon la revendication 1, caractérisé par le fait que $R^1$ et $R^2$ représentent méthyle, X étant S et $R^3$ 2-bromophényle.

10. Composé selon la revendication 1, caractérisé par le fait que $R^1$ et $R^2$ représentent méthyle, X étant S et $R^3$ 3-bromophényle.